(19) Europäisches Patentamt
European Patent Office
Office européen des brevets

(11) Veröffentlichungsnummer: **0 268 295 B1**

(12) **EUROPÄISCHE PATENTSCHRIFT**

(45) Veröffentlichungstag der Patentschrift: **07.10.92**

(51) Int. Cl.5: **C07D 403/04**, C07D 409/14, A01N 47/36

(21) Anmeldenummer: **87117076.7**

(22) Anmeldetag: **19.11.87**

# (54) N-Arylsulfonyl-N'-pyrimidyl-(triazinyl)-harnstoffe.

(30) Priorität: **20.11.86 DE 3639563**

(43) Veröffentlichungstag der Anmeldung:
**25.05.88 Patentblatt 88/21**

(45) Bekanntmachung des Hinweises auf die Patenterteilung:
**07.10.92 Patentblatt 92/41**

(84) Benannte Vertragsstaaten:
**DE FR GB IT**

(56) Entgegenhaltungen:
**EP-A- 0 152 378**
**DE-A- 2 014 983**
**DE-A- 2 028 167**

**CHEMICAL ABSTRACTS, Band 105, Nr. 18, 3. November 1986, Columbus, Ohio, USA; AYYANGAR N.R. et al.: "Synthesis of monoazo disperse dyes from 5-amino-3-methyl-1-(3',5'-disubstituted) s-triazinylpyrazoles and a study of their visible absorption and dyeing properties", Seite 81, Spalte 1, Zusammenfassung-Nr. 154656s**

(73) Patentinhaber: **BASF Aktiengesellschaft**
**Carl-Bosch-Strasse 38**
**W-6700 Ludwigshafen(DE)**

(72) Erfinder: **Eicken, Karl, Dr.**
**Am Huettenwingert 12**
**W-6706 Wachenheim(DE)**
Erfinder: **Plath, Peter, Dr.**
**Hans-Balcke-Strasse 13**
**W-6710 Frankenthal(DE)**
Erfinder: **Wuerzer, Bruno, Dr.**
**Ruedigerstrasse 13**
**W-6701 Otterstadt(DE)**
Erfinder: **Meyer, Norbert, Dr.**
**Dossenheimer Weg 22**
**W-6802 Ladenburg(DE)**

**EP 0 268 295 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte N-Arylsulfonyl-N'-pyrimidyl(triazinyl)-harnstoffe, Verfahren zu ihrer Herstellung und ihre Verwendung zur Bekämpfung unerwünschten Pflanzenwuchses. Darüber hinaus betrifft die Erfindung auch als Zwischenprodukte hergestellte neue Aminopyrimidine und Aminotriazine.

Aus einer Reihe von Patentanmeldungen ist bekannt, daß Sulfonylharnstoffe herbizid wirksam sind. Weiterhin ist bekannt, daß Sulfonylharnstoffe, die im Pyrimidinrest Dimethylamino-Gruppen tragen, herbizid unwirksam sind (G. Levitt in Miyamoto und Kearney in Pesticide Chemistry Vol. 1, p. 243 ff Proceedings of IUPAC-Congress, Kyoto 1982).

Gemäß EP-A 152 378 werden herbizidwirksame Suflonylharnstoffderivate offenbart, die stets am Stickstoff substituiert sind. Offensichtlich ist das Vorhandensein der Gruppe $CHR^5R^6$, in der $R^6$ einen über S, SO oder $SO_2$ gebundenen Rest oder einen heterocyclischen Rest bedeutet, wesentlich für die Wirksamkeit der offenbarten Verbindungen, denn den Zwischenprodukten, z.B. der Struktur IX, wird keinerlei biologische Wirkung zugeschrieben. Im Hinblick auf diesen Stand der Technik ist daher überraschend, daß Sulfonylharnstoffderivate, die nicht am Stickstoff zweifach substituiert sind und einen der genannten Azolylreste am Pyrimidyl- bzw. Triazinylsubstituenten enthalten, zur Bekämpfung von unerwünschtem Pflanzenwuchs geeignet sind.

Es wurde gefunden, daß N-Arylsulfonyl-N'-pyrimidyl(triazinyl)harnstoffe der Formel

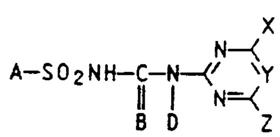

(I),

in der A für einen Rest der Formel

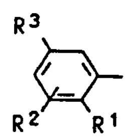 oder 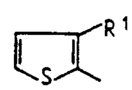 oder 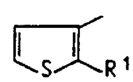

wobei

- $R^1$ Wasserstoff, Halogen, Cyano, Nitro, $C_1$-$C_4$-Halogenalkyl, $C_1$-$C_4$-Alkyl, $C_1$-$C_4$-Alkoxy, CO-$R^4$, S(O)-$_m$-$C_1$-$C_4$-Alkyl oder $SO_2R^5$,
- $R^2$ Wasserstoff, Halogen, Methyl, Ethyl, Methoxy oder Ethoxy,
- $R^3$ Wasserstoff, Halogen, Nitro oder Methoxy,
- $R^4$ Wasserstoff, $C_1$-$C_4$-Alkyl, $C_1$-$C_5$-Alkoxy, $C_3$-$C_5$-Alkenyloxy, $C_3$-$C_5$-Alkinyloxy, $C_1$-$C_5$-Alkylthio, Phenoxy, Benzyloxy oder gegebenenfalls durch 1 bis 3 Halogenatome oder $C_1$-$C_3$-Alkoxy substituiertes $C_1$-$C_5$-Alkoxy,
- $R^5$ gegebenenfalls durch 1 bis 3 Halogenatome substituiertes $C_1$-$C_4$-Alkoxy, Phenoxy oder Benzyloxy und
- m die Zahlen Null, eins oder zwei bedeuten,
- B für Sauerstoff oder Schwefel,
- D für Wasserstoff
- X für $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder Halogen,
- Y für die Methingruppe -CH= oder Stickstoff und
- Z für über Stickstoff gebundenes Pyrazolyl, 1,2,4-Triazolyl, 1,2,3-Triazolyl oder Imidazolyl

stehen, sowie Salze dieser Verbindungen eine vorteilhafte herbizide Wirkung, besonders im Nachauflaufverfahren, haben und gegenüber einer Reihe von Kulturpflanzen selektiv sind.

Besonders bevorzugt sind N-Arylsulfonyl-N'-pyrimidyl(triazinyl)-harnstoffe der Formel I, in der A einen gegebenenfalls substituierten Phenylrest bedeutet, sowie N-Arylsulfonyl-N'-pyrimidyl(triazinyl)-harnstoff der Formel I, in der B Sauerstoff bedeutet.

Als Substituenten der Reste A der allgemeinen Formel I bedeuten je nach C-Zahl: Alkyl beispielsweise Methyl, Ethyl, n-Propyl, i-Propyl, die vier isomeren Butylreste; Alkoxy beispielsweise Methoxy, Ethoxy, n-Propoxy, i-Propoxy, die vier isomeren Butoxyreste, n-Amyloxy, i-Amyloxy, 2-Amyloxy oder 3-Amyloxy, insbesondere Methoxy, Ethoxy oder i-Propoxy; Alkylthio beispielsweise Methylthio, Ethylthio, n-Propylthio, i-Propylthio, n-Butylthio oder n-Pentylthio, insbesondere Methylthio und Ethylthio. Alkenyloxy beispielsweise Allyloxy, Isopropenyloxy, 1-Propenyloxy, 1-Butenyloxy, 2-Butenyloxy, 3-Butenyloxy, 1-Isobutenyloxy, 2-Isobutenyloxy, 1-Pentenyloxy, 2-Pentenyloxy, 3-Pentenyloxy und 4-Pentenyloxy, insbesondere 4-Pentenyl; Alkylsulfinyl beispielsweise Methylsulfinyl, Ethylsulfinyl, n-Propylsulfinyl und n-Butylsulfinyl, insbesondere Methylsulfinyl und Ethylsulfinyl; Alkylsulfonyl beispielsweise Methylsulfonyl, Ethylsulfonyl oder n-Propylsulfonyl, insbesondere Methylsulfonyl und Ethylsulfonyl; Alkinyloxyreste beispielsweise Propargyloxy, 2-Butinyloxy, 3-Butinyloxy sowie isomere Pentinyloxyreste; vorzugsweise Propargyloxy, 2-Butinyloxy oder 3-Butinyloxy.

Halogen als Substituent $R^1$, $R^2$ oder $R^3$ sowie in den Halogenalkyl- oder Halogenalkoxyresten kann Fluor, Chlor und Brom, vorzugsweise Fluor und Chlor, sein.

Die Erfindung umfaßt ebenfalls die Salze, die die N-Arylsulfonyl-N'-pyrimidyl(triazinyl)-harnstoffe der Formel I mit Aminen, Alkali- und Erdalkalimetallbasen oder quaternären Ammoniumbasen bilden können.

Die Verbindungen der Formel I sind nach drei Verfahren erhältlich:

a) Die Verbindungen der Formel I werden erhalten, indem man ein Arylsulfonylisocyanat oder -isothiocyanat der Formel

$A\text{-}SO_2\text{-}N=C=B$ (II),

gegebenenfalls in Gegenwart einer Base, in an sich bekannter Weise mit einem Aminopyrimidin oder -triazin der Formel

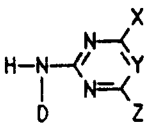

(III)

umsetzt.

b) Nach einem zweiten Verfahren lassen sich die Verbindungen der Formel I durch Umsetzung eines Arylsulfonamids der Formel

$A\text{-}SO_2\text{-}NH_2$ (IV)

mit einem N-Pyrimidyl(triazinyl)-(thio)carbamat der Formel

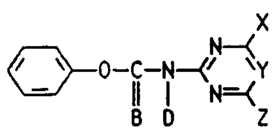

(V)

in Gegenwart einer Base in an sich bekannter Weise herstellen.

c) Weiterhin erhält man die Verbindungen der Formel I durch Umsetzung eines N-Arylsulfonyl(thio)-carbamates der Formel

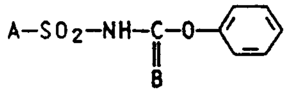

(VI)

mit einem Aminopyrimidin oder -triazin der Formel III in an sich bekannter Weise.

Die nach diesem Verfahren erhaltenen Harnstoffe der Formel I können gewünschtenfalls mittels Aminen, Alkalimetall- oder Erdalkalimetallhydroxiden oder quarternären Ammoniumbasen in Additionssalze übergeführt werden. Dieses geschieht beispielsweise durch Umsetzen mit der äquimolaren Menge Base und Verdampfen des Lösungsmittels.

Alle drei Verfahren werden vorteilhafterweise in aprotischen, inerten, organischen Lösungsmitteln

durchgeführt; geeignet sind Methylenchlorid, Tetrahydrofuran, Acetonitril, Dioxan, Toluol.

Die Reaktionstemperaturen liegen vorzugsweise zwischen -20° und +120° C. Die Umsetzungen verlaufen im allgemeinen leicht exotherm und können gut bei Raumtemperatur durchgeführt werden. Zwecks Abkürzung der Reaktionszeit oder auch zum Einleiten der Umsetzung wird zweckmäßigerweise für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Als Basen können sowohl organische Basen, wie Amine, z.B. Triethylamin, Chinuclidin, Pyridin etc. , als auch anorganische Basen, wie Hydride, z.B. Natrium- oder Calciumhydrid, Hydroxide, z.B. wie Natrium- und Kaliumhydroxid, Carbonate, z.B. Natrium- und Kaliumcarbonat, oder Hydrogencarbonate, z.B. Kalium- und Natriumhydrogencarbonat, verwendet werden.

Die Endprodukte können durch Einengen und/oder Verdampfen des Lösungsmittels isoliert und durch Umkristallisieren oder Zerreiben des festen Rückstandes in Lösungsmitteln, in denen sich die Endprodukte nicht gut lösen (Ether, aromatische Kohlenwasserstoffe oder chlorierte Kohlenwasserstoffe) gereinigt werden, Die Verbindungen der Formel I sind stabile Verbindungen.

Die Zwischenprodukte der Formeln II, IV und VI sind bekannt oder können analog zu bekannten Verfahren hergestellt werden.

Die Aminopyrimidine und Aminotriazine der Formel III sind neu. Sie wurden speziell für die Synthese der Wirkstoffe der Formel I entwickelt und bilden daher einen weiteren Gegenstand der vorliegenden Erfindung.

Die Verbindungen der Formel III werden erhalten, indem man ein Aminopyrimidin oder -triazin der Formel

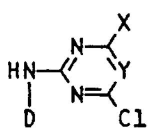

(VII)

in Gegenwart einer Base mit einem Pyrazol, 1,2,4-Triazol, 1,2,3-Triazol und Imidazol oder einem Alkalimetallsalz der genannten Azole umsetzt.

Das Verfahren zur Herstellung der Verbindungen der Formel III wird zweckmäßigerweise in einem inerten polaren Lösungsmittel oder Lösungsmittelgemisch ausgeführt. Geeignete Lösungsmittel sind Ether, wie Dioxan, Tetrahydrofuran, Ethylenglykoldimethylether, Diethylenglykoldimethylether; Alkohole wie Methanol, Ethanol, Ketone wie Aceton, Ethylmethylketon; Dimethylformamid; Acetonitril oder Dimethylsulfoxid.

Als Basen sind in besonderer Weise geeignet: Natrium-und Calciumhydrid, Kalium- und Natriumhydroxid, Kalium- und Natriumcarbonat. In geeigneten Fällen kann die Base in Form einer wäßrigen Lösung zugesetzt werden. Die Menge an Base entspricht mindestens der molaren Menge an Azol.

Die Ausgangsverbindungen der Formel VII sind bekannt oder sind analog zu bekannten Verfahren erhältlich.

Die N-Pyrimidyl(triazinyl)-(thio)carbamate der Formel V sind ebenfalls neu, Sie können analog zu bekannten Verfahren aus den Verbindungen der Formel III hergestellt werden.

Herstellungsbeispiele

1. N-(2-Methoxycarbonylphenyl-sulfonyl)-N'-[4-methyl-6-(1',2',4'-triazol-1'-yl)-pyimid-2-yl]-harnstoff
   a) In eine Lösung von 7,6 g Triazol in 80 ml Dimethylformamid werden 3,5 g Natriumhydrid (80 %) bei 10 ° C in Portionen eingetragen. Nach Ende der Gasentwicklung werden 14,4 g 2-Amino-4-methyl-6-chlor-pyrimidin, gelöst in 60 ml warmem Dimethylformamid, zugegeben und 3 Stdn. bei 100° C gerührt. Nach dem Verdampfen des Lösungsmittels, Anteigen des Rückstands mit Wasser und Umkristallisation aus Dimethylformamid isoliert man 11,8 g 2-Amino-4-methyl-6-(1',2',4'-triazol-1'-yl)-pyrimidin vom Fp. : 278-280° C.
   b) Zu einer Suspension von 6,3 g des obigen Pyrimidins in 70 ml trockenem Tetrahydrofuran und 15 mg Diazabicyclooctan wird eine Lösung von 8,6 g 2-Methoxycarbonylphenylsulfonylisocyanat in 10 ml trockenem Tetrahydrofuran zugetropft. Nach Abklingen der schwach exothermen Reaktion wird über Nacht bei Raumtemperatur nachgerührt. Nach Absaugen und Waschen des Niederschlags mit 10 ml Tetrahydrofuran isoliert man 12,6 g N-(2-Methoxycarbonylphenylsulfonyl)-N'-[4-methyl-6-(1',2',4-triazol-1'-yl)-pyrimid-2-yl]-harnstoff vom Fp: 227 - 230 ° C (Verbindung Nr. 2.09).
2. N-(2-Chlorphenyl-sulfonyl)-N'-(4-pyrazol-1'-yl-6-methoxytriazin-2-yl)-harnstoff

a) In eine Lösung von 6,1 g Pyrazol in 60 ml Dimethylformamid werden 2,7 g Natriumhydrid (80 %) bei 10 °C in Portionen eingetragen und nach Beendigung der Gasentwicklung 13,0 g 2-Amino-4-chlor-6-methoxy-triazin unter Eiskühlung auf einmal zugegeben. Nach Abklingen der schwach exothermen Reaktion wird noch 1 Std. bei 40°C gerührt und dann das Lösungsmittel unter vermindertem Druck entfernt. Aus dem Rückstand isoliert man nach Anteigen mit wenig Eiswasser, Einstellen eines pH-Wert von 4 - 5, Absaugen und Trocknen unter vermindertem Druck 8,0 g 2-Amino-4-pyrazolo-6-methoxy-triazin vom Fp.: 154 - 157 (Z)°C.

b) Zu einer Suspension von 3,9 g des obigen Triazins in 70 ml trockenem Acetonitril und 15 mg Diazabicyclooctan wird eine Lösung von 2-Chlorphenylsulfonylisocyanat in 10 ml trockenem Acetonitril zugetropft. Nach Abklingen der schwach exothermen Reaktion wird 3 Stdn. bei 50 °C gerührt. Nach Abkühlen und Absaugen isoliert man 3,5 g N-(2-Chlorphenylsulfonyl)-N'-(4-pyrazol-1'-yl-6-methoxy-triazin-2-yl)harnstoff vom Fp. : 180-182°C (Verbindung Nr. 2.24).

In analoger Weise lassen sich die in den folgenden Tabellen genannten Verbindungen der Formeln I und III herstellen:

Tabelle 1

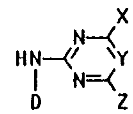

(III)

| Verb.Nr. | D | X | Y | Z | Fp [$^oC$] |
|---|---|---|---|---|---|
| 1.01 | H | $CH_3$ | CH | Pyrazolyl | 155 - 156 |
| 1.02 | H | $OCH_3$ | CH | Pyrazolyl | 115 - 119 |
| 1.03 | H | Cl | CH | Pyrazolyl | 218 - 220 |
| 1.04 | H | $OC_2H_5$ | CH | Pyrazolyl | |
| 1.07 | H | $CH_3$ | CH | 1,2,4-Triazolyl | 278 - 280 |
| 1.08 | H | $OCH_3$ | CH | 1,2,4-Triazolyl | |
| 1.09 | H | $OC_2H_5$ | CH | 1,2,4-Triazolyl | |
| 1.10 | H | Cl | CH | 1,2,4-Triazolyl | |
| 1.13 | H | $CH_3$ | CH | Imidazolyl | 255 (Z) |
| 1.14 | H | $OCH_3$ | CH | Imidazolyl | 223 - 25 |
| 1.15 | H | $OC_2H_5$ | CH | Imidazolyl | |
| 1.16 | H | Cl | CH | Imidazolyl | |
| 1.17 | H | $CH_3$ | N | Pyrazolyl | 188 - 92 |
| 1.18 | H | $OCH_3$ | N | Pyrazolyl | 154 - 157 |
| 1.20 | H | $CH_3$ | N | 1,2,4-Triazolyl | |
| 1.21 | H | $OCH_3$ | N | 1,2,4-Triazolyl | |
| 1.23 | H | $CH_3$ | N | Imidazolyl | |
| 1.24 | H | $OCH_3$ | N | Imidazolyl | 245 (Z) |

Tabelle 2

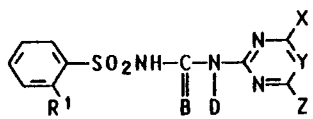

(Ia)

| Verb.Nr. | $R^1$ | B | D | X | Y | Z | Fp [$^oC$] |
|---|---|---|---|---|---|---|---|
| 2.01 | $CO_2CH_3$ | O | H | $CH_3$ | CH | Pyrazolyl | 199 - 202 |
| 2.02 | $CO_2CH_3$ | O | H | $OCH_3$ | CH | Pyrazolyl | 180 - 182 |
| 2.03 | $CO_2CH_3$ | O | H | Cl | CH | Pyrazolyl | |
| 2.05 | $CO_2C_2H_5$ | O | H | $CH_3$ | CH | Pyrazolyl | |
| 2.06 | $CO_2CH_3$ | O | H | $CH_3$ | N | Pyrazolyl | 181 - 183 |
| 2.07 | $CO_2CH_3$ | O | H | $OCH_3$ | N | Pyrazolyl | 158 - 162 |
| 2.09 | $CO_2CH_3$ | O | H | $CH_3$ | CH | 1,2,4-Triazolyl | 227 - 230 |
| 2.10 | $CO_2CH_3$ | O | H | $OCH_3$ | CH | 1,2,4-Triazolyl | |
| 2.11 | $CO_2CH_3$ | O | H | Cl | CH | 1,2,4-Triazolyl | |
| 2.13 | $CO_2CH_3$ | O | H | $CH_3$ | N | 1,2,4-Triazolyl | |
| 2.14 | $CO_2CH_3$ | O | H | $OCH_3$ | N | 1,2,4-Triazolyl | |
| 2.16 | $CO_2CH_3$ | O | H | $CH_3$ | CH | Imidazolyl | 164 - 166 |
| 2.17 | $CO_2CH_3$ | O | H | $OCH_3$ | CH | Imidazolyl | 193 - 196 |
| 2.18 | $CO_2CH_3$ | O | H | $CH_3$ | N | Imidazolyl | |
| 2.19 | $CO_2CH_3$ | O | H | $OCH_3$ | N | Imidazolyl | 170 - 174 |
| 2.20 | Cl | O | H | $CH_3$ | CH | Pyrazolyl | 159 - 163 |

Tabelle 2 (Fortsetzung)

| Verb.Nr. | $R^1$ | B | D | X | Y | Z | Fp [°C] |
|---|---|---|---|---|---|---|---|
| 2.21 | Cl | O | H | $OCH_3$ | CH | Pyrazolyl | 197 - 198 |
| 2.22 | Cl | O | H | Cl | CH | Pyrazolyl | 240 - 243 |
| 2.23 | Cl | O | H | $CH_3$ | N | Pyrazolyl | 205 - 207 |
| 2.24 | Cl | O | H | $OCH_3$ | N | Pyrazolyl | 180 - 182 |
| 2.25 | Cl | O | H | $CH_3$ | CH | 1,2,4-Triazolyl | 206 - 207 |
| 2.26 | Cl | O | H | $OCH_3$ | CH | 1,2,4-Triazolyl | |
| 2.27 | Cl | O | H | $CH_3$ | N | 1,2,4-Triazolyl | |
| 2.28 | Cl | O | H | $OCH_3$ | N | 1,2,4-Triazolyl | |
| 2.29 | Cl | O | H | $CH_3$ | CH | Imidazolyl | 200 - 202 |
| 2.30 | Cl | O | H | $OCH_3$ | CH | Imidazolyl | |
| 2.31 | Cl | O | H | $OCH_3$ | N | Imidazolyl | 194 - 196 |
| 2.32 | $OCH_3$ | O | H | $CH_3$ | CH | Pyrazolyl | |
| 2.33 | $OCH_3$ | O | H | $OCH_3$ | CH | Pyrazolyl | |
| 2.34 | $OCH_3$ | O | H | $OCH_3$ | N | Pyrazolyl | |
| 2.35 | $OCH_3$ | O | H | $CH_3$ | CH | 1,2,4-Triazolyl | |
| 2.36 | $OCH_3$ | O | H | $OCH_3$ | CH | 1,2,4-Triazolyl | |
| 2.37 | $NO_2$ | O | H | $CH_3$ | CH | Pyrazolyl | |
| 2.38 | $NO_2$ | O | H | $OCH_3$ | CH | Pyrazolyl | |
| 2.39 | $NO_2$ | O | H | $OCH_3$ | N | Pyrazolyl | |
| 2.40 | $NO_2$ | O | H | $CH_3$ | CH | 1,2,4-Triazolyl | |
| 2.41 | $SO_2CH_3$ | O | H | $CH_3$ | CH | Pyrazolyl | |
| 2.42 | $SO_2CH_3$ | O | H | $OCH_3$ | CH | Pyrazolyl | |
| 2.43 | $SO_2CH_3$ | O | H | $CH_3$ | CH | 1,2,4-Triazolyl | |
| 2.44 | $CF_3$ | O | H | $OCH_3$ | CH | Pyrazolyl | |
| 2.45 | $CF_3$ | O | H | $CH_3$ | CH | Pyrazolyl | |
| 2.46 | $CF_3$ | O | H | $CH_3$ | CH | 1,2,4-Triazolyl | |

Tabelle 3

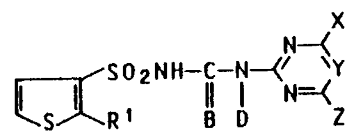

(Ib)

| Verb.Nr. | $R^1$ | B | D | X | Y | Z | Fp.[° C] |
|---|---|---|---|---|---|---|---|
| 3.01 | $CO_2CH_3$ | O | H | $CH_3$ | CH | Pyrazolyl | |
| 3.02 | $CO_2CH_3$ | O | H | $OCH_3$ | CH | Pyrazolyl | |
| 3.03 | $CO_2CH_3$ | O | H | $CH_3$ | N | Pyrazolyl | |
| 3.04 | $CO_2CH_3$ | O | H | $OCH_3$ | N | Pyrazolyl | |
| 3.05 | $CO_2CH_3$ | O | H | $CH_3$ | CH | 1,2,4-Triazolyl | |
| 3.06 | $CO_2CH_3$ | O | H | $OCH_3$ | CH | 1,2,4-Triazolyl | |
| 3.07 | $CO_2CH_3$ | O | H | $CH_3$ | N | 1,2,4-Triazolyl | |
| 3.08 | $CO_2CH_3$ | O | H | $OCH_3$ | N | 1,2,4-Triazolyl | |
| 3.09 | $CO_2CH_3$ | O | H | $CH_3$ | N | Imidazolyl | |
| 3.10 | $CO_2CH_3$ | O | H | $OCH_3$ | CH | Imidazolyl | |

**Tabelle 4**

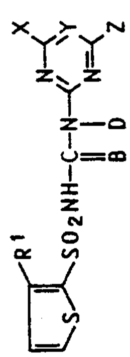

(Ic)

| Verb.Nr. | $R^1$ | B | D | X | Y | Z | Fp.[° C] |
|---|---|---|---|---|---|---|---|
| 4.01 | $CO_2CH_3$ | O | H | $CH_3$ | CH | Pyrazolyl | |
| 4.02 | $CO_2CH_3$ | O | H | $OCH_3$ | CH | Pyrazolyl | |
| 4.03 | $CO_2CH_3$ | O | H | $CH_3$ | N | Pyrazolyl | |
| 4.04 | $CO_2CH_3$ | O | H | $OCH_3$ | N | Pyrazolyl | |
| 4.05 | $CO_2CH_3$ | O | H | $CH_3$ | CH | 1,2,4-Triazolyl | |
| 4.06 | $CO_2CH_3$ | O | H | $OCH_3$ | CH | 1,2,4-Triazolyl | |
| 4.07 | $CO_2CH_3$ | O | H | $CH_3$ | N | 1,2,4-Triazolyl | |
| 4.08 | $CO_2CH_3$ | O | H | $OCH_3$ | N | 1,2,4-Triazolyl | |
| 4.09 | $CO_2CH_3$ | O | H | $CH_3$ | N | Imidazolyl | |
| 4.10 | $CO_2CH_3$ | O | H | $OCH_3$ | CH | Imidazolyl | |

Die N-Arylsulfonyl-N'-pyrimidyl(triazinyl)-harnstoffe der Formel I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden, Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die Applikation der herbiziden Mittel bzw. der Wirkstoffe kann im Vorauflauf- oder vorzugsweise im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 1, vorzugsweise 0,005 bis 0,5 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden.

In Betracht kommen beispielsweise folgende Kulturen:

| Botanischer Name | Deutscher Name |
|---|---|
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |

| Botanischer Name | Deutscher Name |
|---|---|
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Sesamum indicum | Sesam |
| Solanum tuberosum | Kartoffel |
| Sorghum bicolor (s. vulgare) | Mohrenhirse |
| Theobroma cacao | Kakaobaum |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |

Zur Verbreiterung des Wirkungsspektrums und zur Erzielung synergistischer Effekte können die N-Arylsulfonyl-N'-pyrimidyl(triazinyl)-harnstoffe der allgemeinen Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1 ,3-dionderivate und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam zubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate

hergestellt werden.

Anwendungsbeispiele

Die herbizide Wirkung der Verbindungen der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Für Sojapflanzen wird mit Torfmull angereichert, um einen besseren Stand zu erzielen. Die Samen der Testpflanzen wurden nach Arten getrennt flach eingesät.

Die Samen der Testpflanzen werden nach Arten getrennt flach eingesät. Unmittelbar danach werden bei Vorauflaufanwendungen die Wirkstoffe auf die Erdoberfläche aufgebracht. Sie werden hierzu in Wasser als Verteilungsmittel suspendiert oder emulgiert und mittels fein verteilender Düsen gespritzt.

Die Aufwandmenge beträgt dabei 0,06 kg Wirkstoff/ha. Nach dem Aufbringen und Wachstum werden die Gefäße leicht beregnet, um Keimung und Wachstum in Gang zu bringen. Danach werden die Gefäße mit durchsichtigen Plastikhauben abgedeckt, bis die Pflanzen angewachsen sind. Diese Abdeckung bewirkt ein gleichmäßiges Keimen der Testpflanzen, sofern diese nicht durch die Wirkstoffe beeinträchtigt werden.

Für die Nachauflaufbehandlung werden die Testpflanzen je nach Wuchsform erst bis zu einer Wuchshöhe von 3 bis 15 cm angezogen und danach behandelt. Es werden entweder direkt gesäte und in den gleichen Gefäßen aufgewachsene Pflanzen ausgewählt, oder aber sie werden erst als Keimpflanzen in Saatschalen getrennt angezogen und einige Tage vor der Behandlung in die Versuchsgefäße verpflanzt. Eine Abdeckung unterbleibt bei der Nachauflaufbehandlung. Die Aufwandmengen für die Nachauflaufbehandlung variieren und betragen beispielsweise 0,0075, 0,06, 0,125, 0,25 kg Wirkstoff/ha.

Die Versuchsgefäße werden im Gewächshaus aufgestellt, wobei für wärmeliebende Arten wärmere Bereiche (20 bis 35 °C) und für solche gemäßigtere Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Man schätzt dabei visuell den Einfluß der Behandlung im Vergleich zur unbehandelten Kontrolle.

Die in den Gewächshausversuchen verwendeten Pflanzen setzten sich aus folgenden Arten zusammen:

| Latein. Name | Deutscher Name |
|---|---|
| Abutilon theophrasti | Chinesischer Hanf |
| Amarantus retroflexus | Zurückgekrümmter Fuchsschwanz |
| Beta vulgaris | Zuckerrübe |
| Chenopodium album | Weißer Gänsefuß |
| Chrysanthemnum coronarium | Kronenwucherblume |
| Cyperus esculentus | Erdmandel |
| Cyperus iria | - |
| Echinochloa crus-galli | Hühnerhirse |
| Glycine max. | Sojabohne |
| Gossypium hirsutum | Baumwolle |
| Helianthus annuus | Sonnenblumen |
| Lamium amplexicaule | Stengelumfassende Taubnessel |
| Leptochloa fascicularis | - |
| Sesbania exaltata | Turibaum |
| Solanum nigrum | Schwarzer Nachtschatten |
| Sorghum halepense | Mohrenhirse |
| Triticum aestivum | Weizen |
| Veronica spp. | Ehrenpreisarten |
| Zea mays | Mais |

Die Gewächshausversuche zeigen, daß die beispielhaft ausgewählten Wirkstoffe der Formel I bei niedrigen Aufwandmengen eine gute herbizide Wirkung zeigen und sich zur selektiven Bekämpfung von unerwünschten Pflanzen eignen.

Vorauflaufanwendung:

Verbindung Nr. 2.09 bekämpft mit 0,25 kg Wirkstoff/ha unerwünschte grasartige und breitblättrige Pflanzen selektiv, wobei die Kulturpflanzen Soja und Baumwolle nur eine geringe Wuchsbeeinträchtigung erleiden. Der Wirkstoff Nr. 2.31 zeichnet sich beispielsweise durch gute Verträglichkeit für viele Kulturpflanzen aus und eignet sich darum bei Vorauflaufanwendung zur selektiven Bekämpfung von grasartigen Pflanzen.

Nachauflaufanwendung:

Grasartige und breitblättrige Pflanzen werden von Wirkstoff Nr. 2.09 sicher erfaßt, Sojapflanzen hingegen erleiden keinerlei Schädigung.

Der Wirkstoff Nr. 2.07 eignet sich zur Bekämpfung von grasartigen Pflanzen in Baumwolle, während der Wirkstoff Nr. 2.02 eine gute heribzide Wirkung gegen ein breites Spektrum unterschiedlicher Beispielpflanzen entfaltet.

Sauergräser, am Beispiel von Cyperus iria gezeigt, lassen sich mit den Wirkstoffen Nr. 2.02, 2.01, 2.17, 2.06, 2.23 sicher bekämpfen.

Verbindung Nr. 2.31 erfaßt mit 0,125 kg Wirkstoff/ha breitblättrige Unkräuter und Cyperaceen auch selektiv in Sonnenblumen, wobei der Wirkstoff gegenüber dieser Kultur nur geringfügig phytotoxisch wird.

**Patentansprüche**

**1.** N-Arylsulfonyl-N'-pyrimidyl(triazinyl)-harnstoffe der Formel

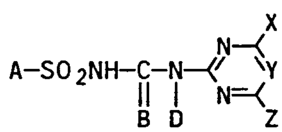 (I),

in der A für einen Rest der Formel

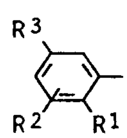 oder 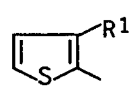 oder 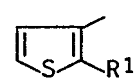

steht, wobei

- $R^1$ Wasserstoff, Halogen, Cyano, Nitro, $C_1$-$C_4$-Halogenalkyl, $C_1$-$C_4$-Alkyl, $C_1$-$C_4$-Alkoxy, CO-$R^4$, $S(O)_m$-$C_1$-$C_4$-Alkyl oder $SO_2R^5$,
- $R^2$ Wasserstoff, Halogen, Methyl, Ethyl, Methoxy oder Ethoxy,
- $R^3$ Wasserstoff, Halogen, Nitro oder Methoxy,
- $R^4$ Wasserstoff, $C_1$-$C_4$-Alkyl, $C_1$-$C_5$-Alkoxy, $C_3$-$C_5$-Alkenyloxy, $C_3$-$C_5$-Alkinyloxy, $C_1$-$C_5$-Alkylthio, Phenoxy, Benzyloxy oder gegebenenfalls durch 1 bis 3 Halogenatome oder $C_1$-$C_3$-Alkoxy substituiertes $C_1$-$C_5$-Alkoxy,
- $R^5$ gegebenenfalls durch 1 bis 3 Halogenatome substituiertes $C_1$-$C_4$-Alkoxy, Phenoxy oder Benzyloxy und
- m die Zahlen Null, eins oder zwei bedeuten,
- B für Sauerstoff oder Schwefel,
- D für Wasserstoff,
- X für $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio oder Halogen,
- Y für die Methingruppe -CH= oder Stickstoff und
- Z für über Stickstoff gebundenes Pyrazolyl, 1,2,4-Triazolyl, 1,2,3-Triazolyl oder Imidazolyl

stehen, sowie Salze dieser Verbindungen.

**2.** N-Arylsulfonyl-N'-pyrimidyl(triazinyl)-harnstoffe der Formel I gemäß Anspruch 1, dadurch gekennzeichnet, daß A einen gegebenenfalls substituierten Phenylrest bedeutet.

3. N-Arylsulfonyl-N'-pyrimidyl(triazinyl)-harnstoffe der Formel I gemäß Anspruch 1, dadurch gekennzeichnet, daß B Sauerstoff bedeutet.

4. Verfahren zur Herstellung der Verbindungen der Formel I gemäß Anspruch 1, dadurch gekennzeichnet, daß man ein Arylsulfonylisocyanat oder der Formel

$A\text{-}SO_2\text{-}N=C=B$ (II),

gegebenenfalls in Gegenwart einer Base, in an sich bekannter Weise mit einem Aminopyrimidin oder -triazin der Formel

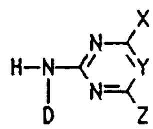

(III)

umsetzt und gegebenenfalls in an sich bekannter Weise in Salze überführt.

5. Verfahren zur Herstellung der Verbindungen der Formel I, dadurch gekennzeichnet, daß man ein Arylsulfonamid der Formel

$A\text{-}SO_2\text{-}NH_2$ (IV)

in Gegenwart einer Base in an sich bekannter Weise mit einem N-Pyrimidyl(triazinyl)-(thio)carbamat der Formel

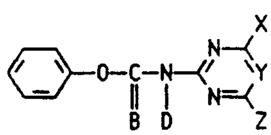

(V)

umsetzt und gegebenenfalls in an sich bekannter Weise in Salze überführt.

6. Verfahren zur Herstellung der Verbindungen der Formel I gemäß Anspruch 1, dadurch gekennzeichnet, daß man ein N-Arylsulfonyl(thio)carbamat der Formel

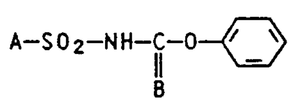

(VI)

in an sich bekannter Weise mit einem Aminopyrimidin oder -triazin der Formel III umsetzt und in an sich bekannter Weise gegebenenfalls in Salze überführt.

7. Aminopyrimidine und -triazine der Formel

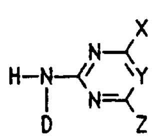

(III),

in der D, X, Y und Z die im Anspruch 1 angegebenen Bedeutungen haben.

8. Verwendung von Aminopyridinen und -triazinen der Formel III gemäß Anspruch 7 zur Herstellung von

N-Arylsulfonyl-N'-pyrimidyl(triazinyl)harnstoffen der Formel I gemäß Anspruch 1.

**9.** Herbizid, enthaltend inerte Zusatzstoffe und einen N-Arylsulfonyl-N'-pyrimidyl(triazinyl)-harnstoff der Formel I gemäß Anspruch 1.

**10.** Verfahren zur Bekämpfung unerwünschten Pflanzenwuchses, dadurch gekennzeichnet, daß man die Pflanzen und/oder ihren Standort mit einer herbizid wirksamen Menge eines N-Arylsulfonyl-N'-pyrimidyl(triazinyl)-harnstoffs der Formel I gemäß Anspruch 1 behandelt.

## Claims

**1.** An N-arylsulfonyl-N'-pyrimidyl(triazinyl)urea of the formula

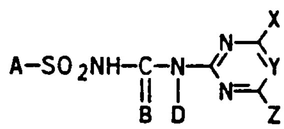
(I),

where A is a radical of the formula

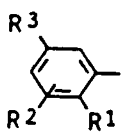
or
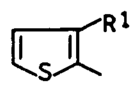
or
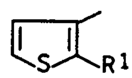

where

- $R^1$ is hydrogen, halogen, cyano, nitro, $C_1$-$C_4$-haloalkyl, $C_1$-$C_4$-alkyl, $C_1$-$C_4$-alkoxy, CO-$R^4$, $S(O)_m$-$C_1$-$C_4$-alkyl or $SO_2R^5$,
- $R^2$ is hydrogen, halogen, methyl, ethyl, methoxy or ethoxy,
- $R^3$ is hydrogen, halogen, nitro or methoxy,
- $R^4$ is hydrogen, $C_1$-$C_4$-alkyl, $C_1$-$C_5$-alkoxy, $C_3$-$C_5$-alkenyloxy, $C_3$-$C_5$-alkynyloxy, $C_1$-$C_5$-alkylthio, phenoxy, benzyloxy or $C_1$-$C_5$-alkoxy which is unsubstituted or substituted by 1 to 3 halogen atoms or $C_1$-$C_3$-alkoxy,
- $R^5$ is $C_1$-$C_4$-alkoxy, phenoxy or benzyloxy, each of which is unsubstituted or substituted by 1 to 3 halogen atoms,
- m is one of the integers 0, 1 and 2,
- B is oxygen or sulfur,
- D is hydrogen,
- X is $C_1$-$C_3$-alkyl, $C_1$-$C_3$-haloalkyl, $C_1$-$C_3$-alkoxy, $C_1$-$C_3$-haloalkoxy, $C_1$-$C_3$-alkylthio or halogen,
- Y is the methine group -CH= or nitrogen and
- Z is pyrazolyl, 1,2,4-triazolyl, 1,2,3-triazolyl or imidazolyl, each of which is linked via nitrogen,

and salts of these compounds.

**2.** An N-arylsulfonyl-N'-pyrimidyl(triazinyl)urea of the formula I as claimed in claim 1, where A is unsubstituted or substituted phenyl.

**3.** An N-arylsulfonyl-N'-pyrimidyl(triazinyl)urea of the formula I as claimed in claim 1, where B is oxygen.

**4.** A process for the preparation of a compound of the formula I as claimed in claim 1, wherein an arylsulfonyl-isocyanate of the formula

$A$-$SO_2$-$N=C=B$ (II)

is reacted with an aminopyrimidine or triazine of the formula

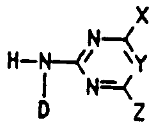

(III)

in a conventional manner in the presence or absence of a base, and the product is, if required, converted into salts in a conventional manner.

**5.** A process for the preparation of a compound of the formula I, wherein an arylsulfonamide of the formula

$A-SO_2-NH_2$ (IV)

is reacted with an N-pyrimidyl(triazinyl)-(thio)carbamate of the formula

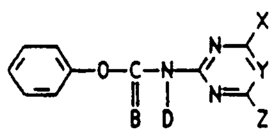

(V)

in a conventional manner in the presence of a base, and the product is, if required, converted into salts in a conventional manner.

**6.** A process for the preparation of a compound of the formula I as claimed in claim 1, wherein an N-arylsulfonyl (thio)carbamate of the formula

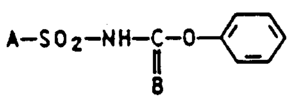

(VI)

is reacted with an aminopyrimidine or triazine of the formula III in a conventional manner, and the product is, if required, converted into salts in a conventional manner.

**7.** An aminopyrimidine and aminotriazine of the formula

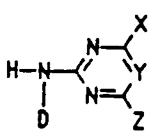

(III)

where D, X, Y and Z have the meanings given in claim 1.

**8.** Use of an aminopyrimidine or -triazine of the formula III as claimed in claim 7 for the preparation of an N-arylsulfonyl-N'-pyrimidyl(triazinyl)urea of the formula I as claimed in claim 1.

**9.** A herbicide containing inert additives and an N-arylsulfonyl-N'-pyrimidyl(triazinyl)urea of the formula I as claimed in claim 1.

**10.** A process for controlling the growth of undesirable plants, wherein the plants and/or their habitat are treated with a herbicidally effective amount of an N-arylsulfonyl-N'-pyrimidyl(triazinyl)urea of the formula I as claimed in claim 1.

**Revendications**

1. N-arylsulfonyl-N'-pyrimidyl(triazinyl)-urées de formule

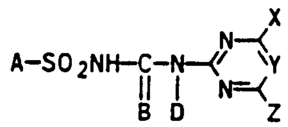 (I),

dans laquelle A représente un groupe de formule

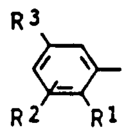 ou 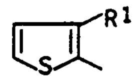 ou 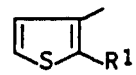

dans lesquelles
$R^1$ représente l'hydrogène, un halogène, un groupe cyano, nitro, halogénoalkyle en C1-C4, alkyle en C1-C4, alcoxy en C1-C4, CO-$R^4$, $S(O)_m$-alkyle en C1-C4 ou $SO_2R^5$,
$R^2$ représente l'hydrogène, un halogène, un groupe méthyle, éthyle, méthoxy ou éthoxy,
$R^3$ représente l'hydrogène, un halogène, un groupe nitro ou méthoxy,
$R^4$ représente l'hydrogène, un groupe alkyle en C1-C4, alcoxy en C1-C5, alcényloxy en C3-C5, alcynyloxy en C3-C5, alkylthio en C1-C5, phénoxy, benzyloxy, ou un groupe alcoxy en C1-C5 éventuellement substitué par 1 à 3 atomes d'halogène ou groupes alcoxy en C1-C5,
$R^5$ représente un groupe alcoxy en C1-C4, phénoxy ou benzyloxy éventuellement substitué par 1 à 3 atomes d'halogène, et
m est égal à 0, 1 ou 2,
B représente l'oxygène ou le soufre,
D représente l'hydrogène,
X représente un groupe alkyle en C1-C3, halogénoalkyle en C1-C3, alcoxy en C1-C3, halogénoalcoxy en C1-C3, alkylthio en C1-C3 ou un halogène,
Y représente le groupe méthine -CH= ou l'azote, et
Z représente un groupe pyrazolyle, 1,2,4-thiozolyle, 1,2,3-triazolyle ou imidazolyle relié par l'azote,
et le sels de ces composés.

2. N-arylsulfonyl-N'-pyrimidyl(triazinyl)-urées de formule I de la revendication 1, caractérisées en ce que A représente un groupe phényle éventuellement substitué.

3. N-arylsulfonyl-N'-pyrimidyl(triazinyl)-urées de formule I de la revendication 1, caractérisées en ce que B représente l'oxygène.

4. Procédé de préparation des composés de formule I de la revendication 1, caractérisé en ce que l'on fait réagir un arylsulfonylisocyanate de formule

$A-SO_2-N=C=B$ (II),

éventuellement en présence d'une base, de manière connue en soi, avec une amino-pyrimidine ou -triazine de formule

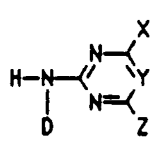 (III)

et le cas échéant on convertit en sels de manière connue en soi.

5. Procédé de préparation des composés de formule I, caractérisé en ce que l'on fait réagir un

arylsulfonamide de formule

A-$SO_2$-$NH_2$ (IV)

en présence d'une base, de manière connue en soi, avec un N-pyrimidyl(triazinyl)-(thio)carbamate de formule

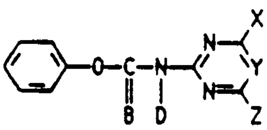

(V)

et éventuellement on convertit en sels de manière connue en soi.

**6.** Procédé de préparation des composés de formule I de la revendication 1, caractérisé en ce que l'on fait réagir un N-arylsulfonyl(thio)carbamate de formule

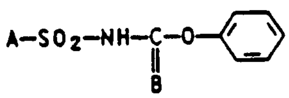

(VI)

de manière connue en soi, avec une amino-pyrimidine ou -triazine de formule III et le cas échéant on convertit en sels de manière connue en soi.

**7.** Amino-pyrimidines et -triazines de formule

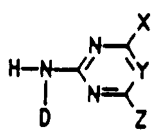

(III),

dans laquelle D, X, Y et Z ont les significations indiquées dans la revendication 1.

**8.** Utilisation des amino-pyridines et -triazines de formule III de la revendication 7 pour la préparation des N-arylsulfonyl-N'-pyrimidyl(triazinyl)-urées de formule I de la revendication 1.

**9.** Produit herbicide contenant des additifs inertes et une N-arylsulfonyl-N'-pyrimidyl(triazinyl)-urée de formule I de la revendication 1.

**10.** Procédé pour combattre les croissances de végétaux indésirables, caractérisé en ce que l'on traite les végétaux et/ou leur habitat par une quantité herbicide efficace d'une N-arylsulfonyl-N'-pyrimidyl-(triazinyl)-urée de formule I de la revendication 1.